# EUROPÄISCHE PATENTSCHRIFT

(11) **EP 0 950 388 B1**
(45) Veröffentlichungstag und Bekanntmachung des Hinweises auf die Patenterteilung: **12.05.2004**
(21) Anmeldenummer: 99102353.2
(22) Anmeldetag: 06.02.1999
(51) Int. Cl.: A61F 2/44, A61F 2/30

(54) **Implantat zum Einsetzen zwischen Wirbelkörper der Wirbelsäule**
Implant for the insertion between the vertebraes of the spine
Implant à inserér entre les vertèbres de la colonne vertébrale

(30) Priorität: 16.04.1998 DE 19816782
(43) Veröffentlichungstag der Anmeldung: 20.10.1999
(73) Patentinhaber: Ulrich GmbH & Co. KG, 89081 Ulm (DE)
(72) Erfinder: Sutcliffe, John, Good Caster, Essex CM 1 4PR (GB)
(74) Vertreter: Hentrich, Swen Dipl.-Phys. Dr.

(56) Entgegenhaltungen:
- WO-A-96/17564
- WO-A-96/37170
- US-A- 5 360 430
- US-A- 5 458 641

## Beschreibung

Die Erfindung betrifft ein Implantat zum Einsetzen zwischen Wirbelkörper der Wirbelsäule als Platzhalter für aus der Wirbelsäule entfernte Wirbel oder Wirbelteile, bestehend aus zwei endständigen, zur Anlage an den angrenzenden Wirbel bestimmten Implantatteilen und einem dazwischen befindlichen, mittigen Implantatteil, wobei an den endständigen Implantatteilen jeweils eine zu dem angrenzenden Wirbel weisenden und diesen zumindest teilweise überdecken, im Längsschnitt L-förmige Fixierungsplatte angeordnet ist.

Ein derartiges Implantat ist aus der US 5 360 430 bekannt, bei dem in den plattenförmigen endständigen Implantatteilen die Fixierungsplatten in einen Schlitz eingeschoben werden können.

Ein weiteres Implantat ist aus der DE 44 23 257 A1 bekannt. Dieses Implantat hat sich in der Praxis bewährt, da es gut geeignet ist, axial wirkende Kräfte zwischen den beiden angrenzenden Wirbeln zu übertragen, wobei aufgrund der Unterteilung zwischen endständigen Implantatteilen und mittigem Implantatteil eine Distraktionsmöglichkeit des Implantats während der Operation gegeben ist. Um der Wirbelsäule aber auch in der durch das Implantat ausgefüllten Lücke zwischen den beiden angrenzenden Wirbeln eine ausreichende Stabilität zu geben, ist es nicht nur erforderlich, axial wirkende Kräfte zu berücksichtigen. Um eine ausreichende Stabilität der gesamten Wirbelsäule zu erzielen wird üblicherweise zusätzlich zu dem Implantat eine den Platzhalter übergreifende Platte oder ein Stab bzw. eine dorsale Instrumentierung an den beiden angrenzenden Wirbeln mittels Knochenschrauben befestigt. Ein vergleichbares Implantat ist auch in WO 96/37 170 offenbart.

Aufgabe der Erfindung ist es, ein Implantat der eingangs genannten Art so weiterzubilden, daß eine den Platzhalter verdeckende, Radial- und Scherkräfte aufnehmende Platte oder ein Stab bzw. eine dorsale Instrumentierung verzichtbar ist.

Diese Aufgabe wird nach der Erfindung bei einem Implantat der eingangs genannten Art dadurch gelöst, daß die endständigen Implantatteile durch je ein Gewinde mit dem mittigen Implantatteil verbunden sind und dieses außenseitig übergreifen, und daß dieFixierungsplatte an dem kurzen Schenkel zur lösbaren Anlage an das zugeordnete endständige Implantatteil einen kreisbogenförmigen Bund aufweist.

Das erfindungsgemäße Implantat bietet den Vorteil, daß der Raum zwischen den beiden angrenzenden Wirbeln zugänglich bleibt, so daß zum einen auch in späteren Stadien der Operation Korrekturen bezüglich der Distraktion vorgenommen werden können, und daß zum anderen die Implantatteile selber frei zugänglich sind, insbesondere die Außenflächen der endständigen Implantatteile, die endständigen Implantatteile das mittige Implantatteil außenseitig übergreifen, um diese beispielsweise mit Knochenzement oder patienteneigenen oder fremden Knochenspänen auszufüllen und besser einwachsen zu lassen, wobei durch die positionierten Fixierungsplatten eine bessere Stabilität des Implantats und damit der Wirbelsäule erreicht ist.

Zweckmäßigerweise ist die Fixierungsplatte zur Anpassung an die Krümmung des Wirbels im Querschnitt konvex geformt, so daß eine relativ große Anlagefläche zwischen Fixierungsplatte und Wirbel vorliegt.

Um eine individuelle Anpassung an die während der Operation vorgefundenen Gegebenheiten zu ermöglichen, ist in der Fixierungsplatte ein parallel zur Längsachse der Wirbelsäule ausgerichtetes Langloch ausgebildet, in dem eine Knochenschraube verschoben werden kann, bis sie die richtige Stellung zur Befestigung an dem Wirbel aufweist.

Dabei hat es sich als günstig erwiesen, wenn in dem Langloch Rastsitze für die Aufnahme des Schraubenkopfes der Knochenschraube angeordnet sind, so daß der Schraubenkopf in der Fixierungsplatte versenkt werden kann und gegenüber dieser nicht nur durch Reibung, sondern durch einen Formschluß gehalten bzw. zur Fixierungsplatte axial festgelegt ist.

Nach einer besonders bevorzugten Ausführungsform ist weiterhin vorgesehen, daß an den beiden Seiten des Bundes der Fixierungsplatte Öffnungen ausgebildet sind, die sich mit zwei Aufnahmen in dem zugeordneten endständigen Implantatteil decken und in die Gewindebolzen einschraubbar sind. Diese Ausführungsform bietet den Vorteil, daß die endständigen Implantatteile und die Fixierungsplatte nicht als einstückige Bauteile ausgebildet sein müssen, so daß die Fixierungsplatte nachträglich an dem endständigen Implantatteil befestigt werden kann, die Anlage des endständigen Implantatteils an den angrenzenden Wirbel damit besser beobachtbar, zugänglich und damit auch leichter einstellbar oder aber gänzlich alleinstehend durchführbar ist.

Bei einer weiteren Ausführungsform ist vorgesehen, daß die Fixierungsplatte in Längsrichtung einen Schlitz aufweist, der mittels einer senkrecht zu dem Schlitz orientierten Schraube zur Ausbildung einer Klemmung der Bolzen in den Aufnahmen in seiner Weite verstellbar ist.

Eine Verstellung der Weite des Schlitzes läßt sich ohne große Kraft dadurch erreichen, daß der Schlitz auf der dem mittigen Implantatteil abgewandten Seite durch eine Bohrung begrenzt ist. Durch die Bohrung bildet die Fixierungsplatte eine Art Klammer, deren beiden Schenkel zusammengedrückt werden können, ohne daß es am Ende des Schlitzes zu einer starken Kompression des Materials kommt.

Eine bessere Fixierung der Fixierungsplatte am Wirbel läßt sich dadurch erreichen, daß beidseits des Schlitzes ein Langloch angeordnet ist, durch das je eine Knochenschraube durchgeführt werden kann.

Im Rahmen der Erfindung ist weiterhin vorgesehen, daß die Wände der Implantatteile von Aussparungen durchbrochen sind, so daß die Implantatteile einen Hohlraum umschließen, der zum besseren Einwachsen des Implantats mit Knochenzement oder ähnlichem ausgefüllt werden kann.

Im folgenden soll die Erfindung an einem in der Zeichnung dargestellten Ausführungsbeispiel näher erläutert werden; es zeigen:

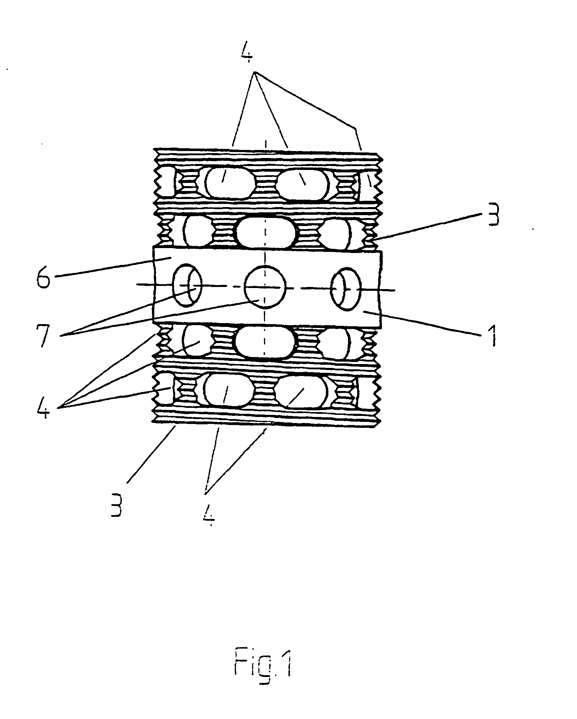

- Fig. 1: das als rohrförmige Hülse ausgebildete mittige Implantatteil mit gegenläufigen Drehsinn aufweisenden Gewinden,

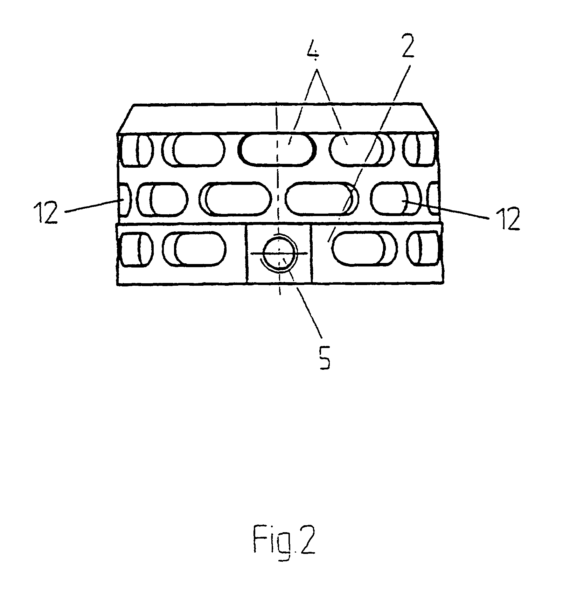

- Fig. 2: ein endständiges Implantatteil,

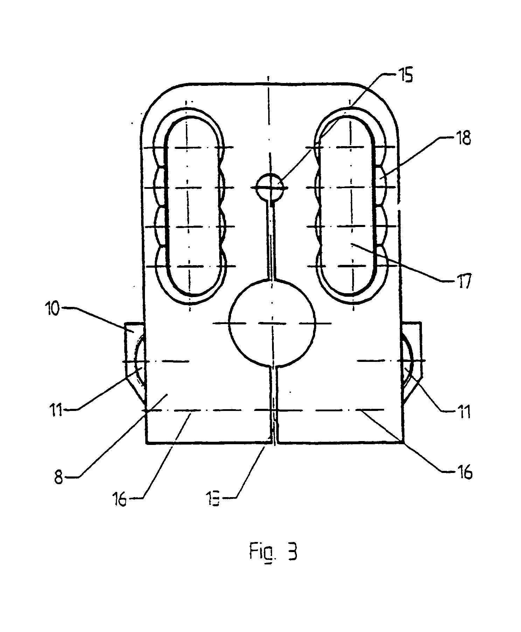

- Fig. 3: eine Fixierungsplatte in einer Vorderansicht,
- Fig. 4: die Fixierungsplatte in einer Seitenansicht,

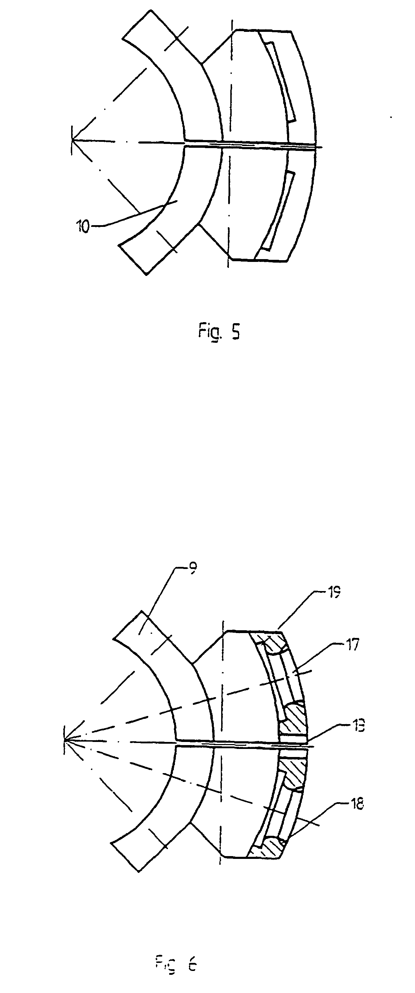

- Fig. 5: die Fixierungsplatte in einer Draufsicht,
- Fig. 6: der Schnitt VI - VI aus Fig. 4,
- Fig. 7: der Schnitt VII - VII aus Fig. 4,
- Fig. 8: der Schnitt VIII - VIII aus Fig. 4,
- Fig. 9: eine Seitenansicht zweier benachbarter Wirbel mit einem zwischen ihnen eingesetzten Implantat, und
- Fig. 10: eine Sicht aus Richtung des Pfeiles X aus Fig. 9.

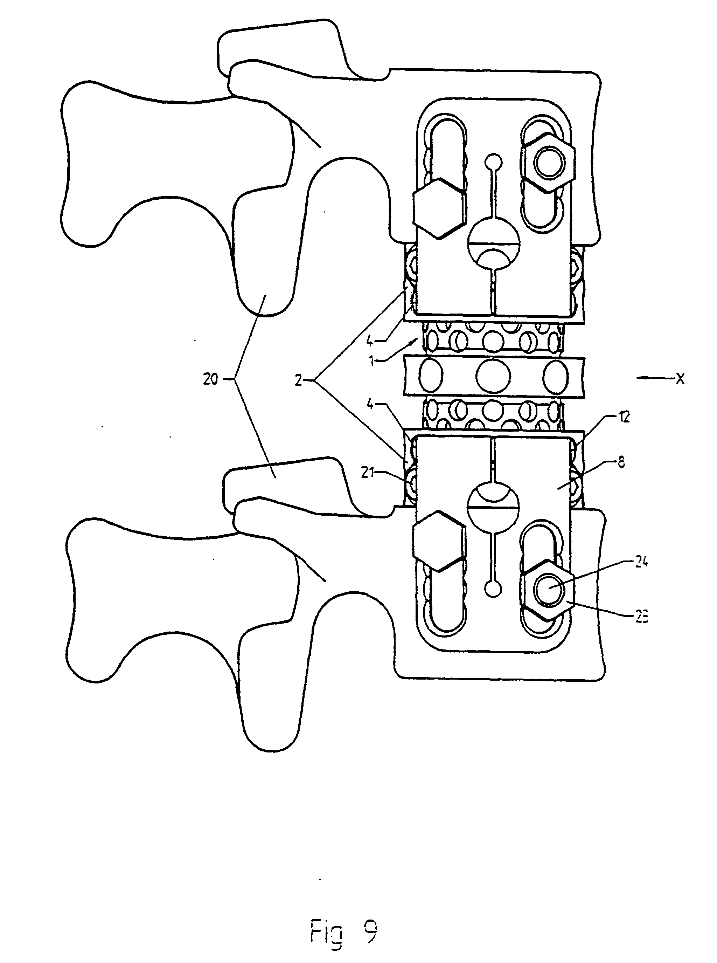

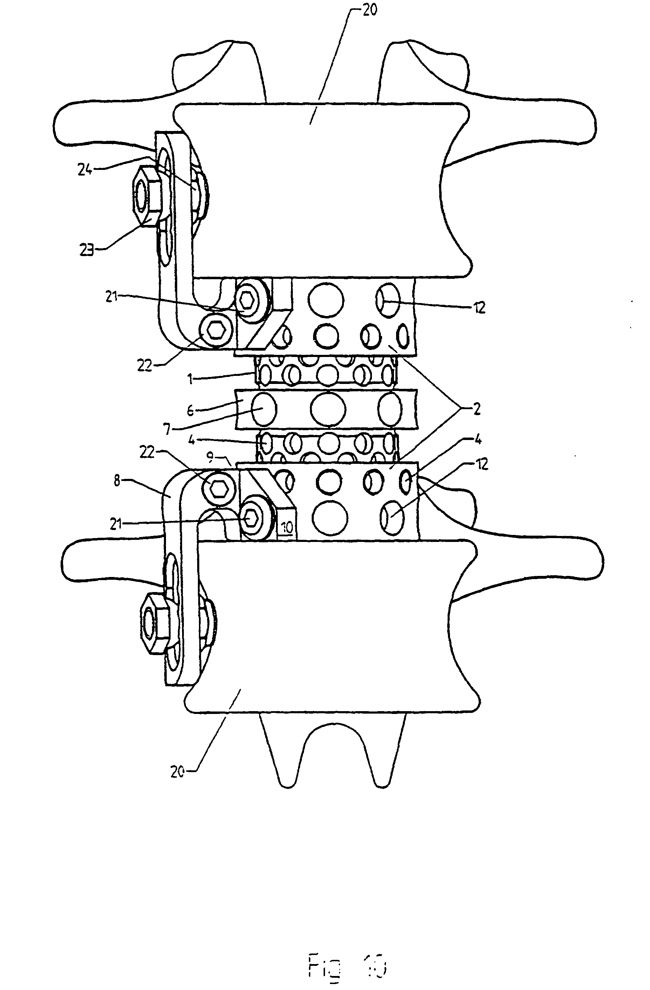

Das in den Fig. 9 und 10 dargestellte Implantat dient zum Einsetzen zwischen Wirbelkörper 20 der Wirbelsäule als Platzhalter für aus der Wirbelsäule entfernte Wirbel oder Wirbelteile. Das Implantat besteht aus einem mittigen Implantatteil 1 und zwei endständigen Implantatteilen 2, wobei das mittige Implantatteil 1 hülsenförmig ausgebildet ist und an seinen Enden je ein Gewinde 3 aufweist, die einen zueinander gegenläufigen Gewindesinn besitzen. Die endständigen Implantatteile 2 sind gleichfalls hülsenförmig ausgebildet mit kreisförmigem Querschnitt. Die endständigen Implantatteile 2 weisen ein Innengewinde auf, mit dem diese auf das Gewinde 3 des mittigen Implantatteils 1 aufgeschraubt werden können. Die Wände des mittigen Implantatteils 1 und der endständigen Implantatteile 2 sind von Aussparungen 4 durchbrochen. Weiterhin verfügen die endständigen Implantatteile 2 über Gewindebohrungen 5, in denen über eine Schraube die axiale Lage der endständigen Implantatteile 2 gegenüber den mittigen Implantatteilen 1 über Klemmung fixiert werden kann. Das mittige Implantatteil 1 weist einen Ringbund 6 auf, in dem Öffnungen 7 ausgebildet sind, in die ein Werkzeug eingeführt werden kann, um das mittige Implantatteil 1 gegenüber den endständigen Implantatteilen 2 zu verdrehen und so entsprechend dem Drehsinn eine Aufweitung im Sinne einer Distraktion oder eine Verengung des Implantats ermöglicht ist.

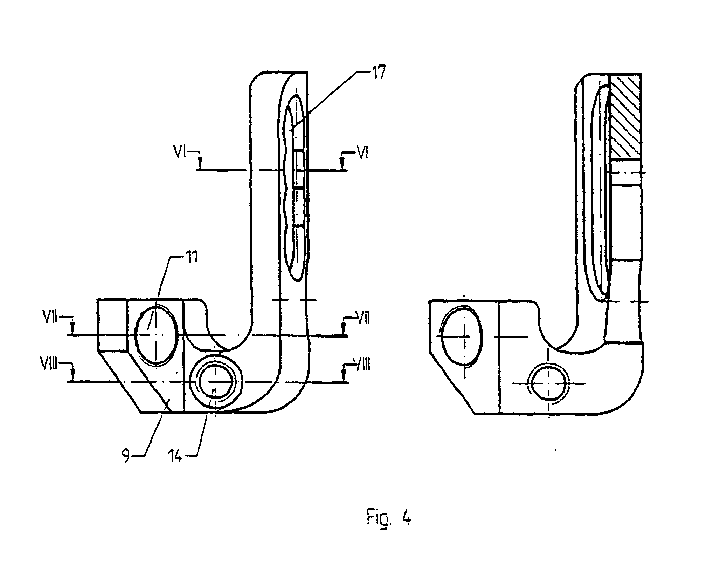

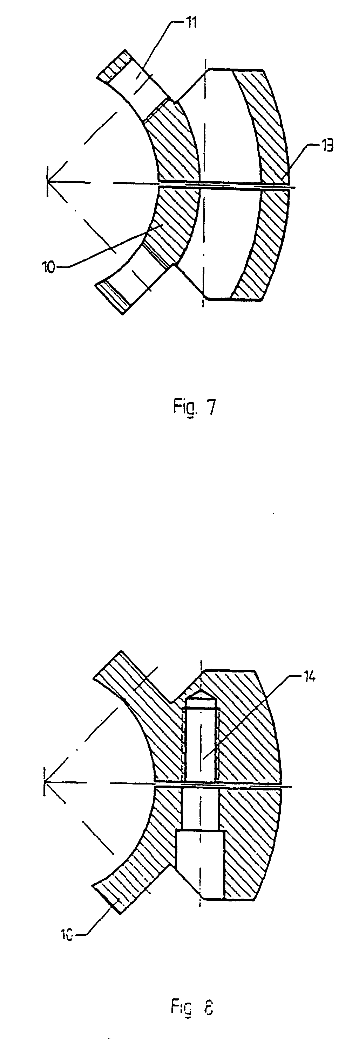

An den endständigen Implantatteilen 2 ist eine jeweils zu dem angrenzenden Wirbel weisende Fixierungsplatte 8 angeordnet, die den angrenzenden Wirbelkörper 20 teilweise überdeckt. Diese Fixierungsplatte 8 ist im Längsschnitt L-förmig (Fig. 4) und im Querschnitt konvex gekrümmt (Fig. 6-8) ausgebildet. Die Fixierungsplatte 8 besitzt an dem kurzen Schenkel 9 ihrer L-förmigen Gestalt einen kreisbogenförmigen Bund 10, der an dem endständigen Implantatteil 2 zur Anlage kommt, wobei zur Befestigung der Fixierungsplatte 8 an dem endständigen Implantatteil 8 an den beiden Seiten des Bundes 10 Öffnungen 11 ausgebildet sind, die mit zwei in dem endständigen Implantatteil 2 ausgebildeten Aufnahmen 12 zur Deckung gebracht werden können. Durch die Öffnungen 11 sind in die Aufnahmen 12 Bolzen 21 einsteckbar. Ein fester Sitz wird dadurch erreicht, daß die Fixierungsplatte 8 in Längsrichtung einen Schlitz 13 aufweist, der in seiner Weite verstellbar ist, so daß eine Klemmung der Bolzen 21 in den Aufnahmen 12 erreicht wird. Zur Veränderung der Weite des Schlitzes 13 ist am Schnittpunkt der beiden Schenkel der L-förmigen Fixierungsplatte 8 eine senkrecht zu dem Schlitz 13 orientierte Gewindebohrung 14 vorhanden, in die eine Schraube 22 eingeschraubt werden kann, so daß die beiden beidseits des Schlitzes 13 der Fixierungsplatte 8 angeordneten Schenkel 16 gegeneinander verstellt werden können. Der Schlitz 13 ist auf der dem mittigen Implantatteil 1 abgewandten Seite durch eine Bohrung 15 begrenzt, die die Verstellung der beiden Schenkel 16 gegeneinander erleichtert. Nach einer alternativen, in der Zeichnung nicht gezeigten Ausführungsform sind die Bolzen als Gewindebolzen ausgebildet, die in die mit einem Gewinde versehenen Aufnahmen (12) einschraubbar sind.

Beidseits des Schlitzes 13 ist in den Schenkeln 16 jeweils ein Langloch 17 ausgebildet, in dem Rastsitze 18 für die Aufnahme des Schraubenkopfes 23 von Knochenschrauben 24 angeordnet sind. An der dem Wirbelkörper 20 zugewandten Seite jedes Langloches 17 ist eine Hinterfräsung 19 ausgebildet.

## Patentansprüche

1. Implantat zum Einsetzen zwischen Wirbelkörper der Wirbelsäule als Platzhalter für aus der Wirbelsäule entfernte Wirbel oder Wirbelteile, bestehend aus zwei endständigen, zur Anlage an den angrenzenden Wirbel bestimmten Implantatteilen (2) und einem dazwischen befindlichen, mittigen Implantatteil (1), wobei an den endständigen Implantatteilen (2) jeweils eine zu dem angrenzenden Wirbel weisende und diesen zumindest teilweise überdeckende, im Längsschnitt L-förmige Fixierungsplatte (8) angeordnet ist, **dadurch gekennzeichnet, daß** die endständigen Implantatteile (2) durch je ein Gewinde (3) mit dem mittigen Implantatteil (1) verbunden sind und dieses außenseitig übergreifen, und daß die Fixierungsplatte (8) an dem kurzen Schenkel (9) zur lösbaren Anlage an das zugeordnete endständige Implantatteil (2) einen kreisbogenförmigen Bund (10) aufweist.

2. Implantat nach Anspruch 1, **dadurch gekennzeichnet, daß** die Fixierungsplatte (8) zur Anpassung an die Krümmung des Wirbels im Querschnitt konvex gekrümmt ist.

3. Implantat nach Anspruch 1 oder 2, **dadurch gekennzeichnet, daß** in der Fixierungsplatte (8) ein parallel zur Längsachse der Wirbelsäule ausgerichtetes Langloch (17) ausgebildet ist.

4. Implantat nach Anspruch 3, **dadurch gekennzeichnet, daß** in dem Langloch (17) Rastsitze (18) für die Aufnahme des Schraubenkopfes einer Knochenschraube angeordnet sind.

5. Implantat nach einem der Ansprüche 1 bis 4, **dadurch gekennzeichnet, daß** an den beiden Seiten des Bundes (10) der Fixierungsplatte (8) Öffnungen (11) ausgebildet sind, die sich mit zwei Aufnahmen (12) in dem zugeordneten endständigen Implantatteil (2) decken und in die Gewindebolzen einschraubbar sind.

6. Implantat nach Anspruch 5, **dadurch gekennzeichnet, daß** in die Aufnahmen (12) Bolzen einsteckbar sind, und daß die Fixierplatte (8) in Längsrichtung (13) einen Schlitz aufweist, der mittels einer senkrecht zu dem Schlitz (13) orientierten Schraube zur Ausbildung einer Klemmung der Bolzen in den Aufnahmen (12) in seiner Weite verstellbar ist.

7. Implantat nach Anspruch 6, **dadurch gekennzeichnet, daß** der Schlitz (13) auf der dem mittigen Implantatteil (1) abgewandten Seite durch eine Bohrung (15) begrenzt ist.

8. Implantat nach Anspruch 6 oder 7, **dadurch gekennzeichnet, daß** beidseits des Schlitzes (13) ein Langloch (17) angeordnet ist.

9. Implantat nach einem der Ansprüche 1 bis 8, **dadurch gekennzeichnet, daß** die Wände der Implantatteile (1, 2) von Aussparungen (4) durchbrochen sind.

## Claims

1. Implant for deployment between vertebrae in the spine, as a place holder for vertebrae or parts of vertebrae that have been removed from the spine,
comprising two terminal implant parts (2) which are intended to rest against the adjacent vertebrae, and a central implant part (1) located between them, wherein arranged on the terminal implant parts (2) there is respectively a fixing plate (8) which points towards the adjacent vertebra and overlaps it at least partially, and which has an L-shaped longitudinal cross-section,
**characterised in that**
the terminal implant parts (2) are connected to the central implant part (1) by means of a screw thread (3) in each case, and that they overlap it on the outside, and that on the short leg (9) the fixing plate (8) has a shoulder (10) in the shape of a circular arc, to rest detachably on the assigned terminal implant part (2).

2. Implant in accordance with claim 1, **characterised in that** for adaptation to the curvature of the spine, the cross-section of the fixing plate (8) is curved in a convex manner.

3. Implant in accordance with claim 1 or 2, **characterised in that** formed in the fixing plate (8) is a long hole (17) that is aligned parallel to the longitudinal axis of the spine.

4. Implant in accordance with claim 3, **characterised in that** arranged in the long hole (17) are catch seats (18) for receiving the screw head of a bone screw.

5. Implant in accordance with one of the claims 1 to 4, **characterised in that** formed on the two sides of the shoulder (10) of the fixing plate (8) are openings (11) which are congruent with two seats (12) in the assigned end implant part (2), and into which threaded bolts can be screwed.

6. Implant in accordance with claim 5, **characterised in that** bolts can be inserted into the seats (12), and that in the longitudinal direction (13) the fixing plate (8) has a slit that can be adjusted in terms of its width by means of a screw oriented perpendicular to the slit (13), to effect jamming of the bolts in the seats (12).

7. Implant in accordance with claim 6, **characterised in that** the slit (13) on the side facing away from the central implant part (1) is limited by a drilled hole (15).

8. Implant in accordance with claim 6 or 7, **characterised in that** a long hole (17) is arranged on both sides of the slit (13).

9. Implant in accordance with one of the claims 1 to 8, **characterised in that** the walls of the implant parts (1, 2) are punctuated by cut-outs (4).

## Revendications

1. Implant destiné à être inséré entre des vertèbres de la colonne vertébrale en tant qu'élément de maintien d'écartement pour des vertèbres ou des parties de vertèbre retirées de la colonne vertébrale, comprenant deux éléments d'implant (2) d'extrémité, destinés à venir en appui sur la vertèbre contiguë et un élément d'implant (1) médian disposé entre deux, une plaque de fixation (8) à profil en L vu en coupe longitudinale qui est tournée vers la vertèbre contiguë et recouvre au moins partiellement celle-ci étant disposée sur les éléments d'implant (2) d'extrémité, **caractérisé par le fait que** les éléments d'implant (2) d'extrémité sont liés chaque fois par un filetage (3) à l'élément d'implant (1) médian et recouvrent celui-ci extérieurement, et **par le fait que** la plaque de fixation (8) au niveau de son aile (9) courte présente un épaulement (10) en arc de cercle destiné à venir en appui démontable sur l'élément d'implant (2) d'extrémité concerné.

2. Implant selon la revendication 1, **caractérisé par le fait que** la plaque de fixation (8), à des fins d'adaptation à la courbure de la vertèbre présente en coupe transversale une courbure convexe.

3. Implant selon la revendication 1 ou 2, **caractérisé par le fait qu'**un trou oblong (17) orienté parallèlement à l'axe longitudinal de la colonne vertébrale est aménagé dans la plaque de fixation (8).

4. Implant selon la revendication 3, **caractérisé par le fait que** des logements d'encliquetage (18) destinés à recevoir la tête d'une vis à os sont disposés dans le trou oblong (17).

5. Implant selon une des revendications 1 à 4, **caractérisé par le fait que** de part et d'autre de l'épaulement (10) de la plaque de fixation (8) sont aménagées des ouvertures (11) qui coïncident avec deux logements . (12) dans l'élément d'implant (2) d'extrémité concerné et dans lesquelles des vis peuvent être vissées.

6. Implant selon la revendication 5, **caractérisé par le fait que** des axes peuvent être insérés dans les logements (12) et **par le fait que** la plaque de fixation (8) dans la direction longitudinale (13) présente une fente dont la largeur peut être modifiée à l'aide d'une vis implantée perpendiculairement à la fente (13) aux fins de former un blocage de l'axe dans les logements (12).

7. Implant selon la revendication 6, **caractérisé par le fait que** la fente (13) du côté éloigné de l'élément d'implant (1) médian est limitée par un trou (15).

8. Implant selon la revendication 6 ou 7, **caractérisé par le fait qu'**un trou oblong (17) est disposé de part et d'autre de la fente (13).

9. Implant selon une des revendications 1 à 8, **caractérisé par le fait que** les parois des éléments d'implant (1, 2) sont percées d'évidements (4).
